# EUROPEAN PATENT SPECIFICATION

(11) **EP 2 463 385 B1**
(45) Date of publication and mention of the grant of the patent: **27.08.2014**
(21) Application number: 11193984.9
(22) Date of filing: 13.11.2009
(51) Int. Cl.: C12Q 1/68

(54) **Kit for RNA detection**
Kit zur RNA-Detektion
Kit pour la détection d'ARN

(30) Priority: 13.11.2008 EP 08019859
(43) Date of publication of application: 13.06.2012
(62) Divisional of application: 09752181.9
(73) Proprietor: RiboxX GmbH, 01445 Radebeul (DE)
(72) Inventor: Rohayem, Jacques, 01277 Dresden (DE)
(74) Representative: Habermann, Hruschka & Schnabel

(56) References cited:
- EP-A2- 1 318 203
- CHO M W ET AL: "RNA duplex unwinding activity of poliovirus RNA-dependent RNA polymerase 3Dpol.", JOURNAL OF VIROLOGY JUN 1993, vol. 67, no. 6, June 1993 (1993-06), pages 3010-3018, XP002563519, ISSN: 0022-538X
- ROHAYEM JACQUES ET AL: "Protein-primed and de novo initiation of RNA synthesis by norovirus 3Dpol.", JOURNAL OF VIROLOGY JUL 2006, vol. 80, no. 14, July 2006 (2006-07), pages 7060-7069, XP002575656, ISSN: 0022-538X
- FULLERTON STEPHEN W B ET AL: "Structural and functional characterization of sapovirus RNA-dependent RNA polymerase", JOURNAL OF VIROLOGY, THE AMERICAN SOCIETY FOR MICROBIOLOGY, US, vol. 81, no. 4, 1 February 2007 (2007-02-01), pages 1858-1871, XP002544505, ISSN: 0022-538X, DOI: 10.1128/JVI.01462-06

### SEQUENCE LISTING

<110> Riboxx GmbH
<120> RNA detection method
<130> R 0057 WOEPT1
<150> 08019859.1 <151> 2008-11-13
<160> 10
<170> PatentIn version 3.3
<210> 1
   <211> 520
   <212> PRT
   <213> Norovirus
<400> 1
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145                 150                 155                 160
   Val Tyr Thr Gly Ala Leu Lys Asp Glu Leu Val Lys Thr Asp Lys Ile
                   165                 170                 175
   Tyr Gly Lys Ile Lys Lys Arg Leu Leu Trp Gly Ser Asp Leu Ala Thr
               180                 185                 190
   Met Ile Arg Cys Ala Arg Ala Phe Gly Gly Leu Met Asp Glu Leu Lys
           195                 200                 205
   Ala His Cys Val Thr Leu Pro Ile Arg Val Gly Met Asn Met Asn Glu
       210                 215                 220
   Asp Gly Pro Ile Ile Phe Glu Arg His Ser Arg Tyr Lys Tyr His Tyr
   225                 230                 235                 240
   Asp Ala Asp Tyr Ser Arg Trp Asp Ser Thr Gln Gln Arg Ala Val Leu
                   245                 250                 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465 470 475 480
   Pro Met Phe Arg Trp Met Arg Phe Ser Asp Leu Ser Thr Trp Glu Gly
   485 490 495
   Asp Arg Asn Leu Ala Pro Ser Phe Val Asn Glu Asp Gly Val Glu Val
   500 505 510
   Asp Lys Leu Ala Ala Ala Leu Glu
   515 520
<210> 2
   <211> 517
   <212> PRT
   <213> Sapovirus
<400> 2
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465 470 475 480
   Thr Asn Tyr Asp Gln Ala Leu Ala Thr Tyr Asn Ala Trp Phe Ile Gly
   485 490 495
   Gly Thr Val Pro Asp Pro Val Gly His Thr Glu Gly Thr His Lys Ile
   500 505 510
   Val Phe Glu Met Glu
   515
<210> 3
   <211> 533
   <212> PRT
   <213> Vesivirus
<400> 3
   Met Lys Val Thr Thr Gln Lys Tyr Asp Val Thr Lys Pro Asp Ile Ser
   1 5 10 15
   Tyr Lys Gly Leu Ile Cys Lys Gln Leu Asp Glu Ile Arg Val Ile Pro
   20 25 30
   Lys Gly Thr Arg Leu His Val Ser Pro Ala His Thr Asp Asp Tyr Asp
   35 40 45
   Glu Cys Ser His Gln Pro Ala Ser Leu Gly Ser Gly Asp Pro Arg Cys
   50 55 60
   Pro Lys Ser Leu Thr Ala Ile Val Val Asp Ser Leu Lys Pro Tyr Cys
   65 70 75 80
   Glu Lys Thr Asp Gly Pro Pro His Asp Ile Leu His Arg Val Gln Arg
   85 90 95
   Met Leu Ile Asp His Leu Ser Gly Phe Val Pro Met Asn Ile Ser Ser
   100 105 110
   Glu Pro Ser Met Leu Ala Ala Phe His Lys Leu Asn His Asp Thr Ser
   115 120 125
   Cys Gly Pro Tyr Leu Gly Gly Arg Lys Lys Asp His Met Ile Gly Gly
   130 135 140
   Glu Pro Asp Lys Pro Leu Leu Asp Leu Leu Ser Ser Lys Trp Lys Leu
   145 150 155 160
   Ala Thr Gln Gly Ile Gly Leu Pro His Glu Tyr Thr Ile Gly Leu Lys
   165 170 175
   Asp Glu Leu Arg Pro Val Glu Lys Val Gln Glu Gly Lys Arg Arg Met
                   180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                     240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
           275                 280                 285
   Leu Lys Ser Pro Pro Ile Ala Ile Phe Asn Gly Val Ala Val Lys Val
       290                 295                 300
   Thr Ser Gly Leu Pro Ser Gly Met Pro Leu Thr Ser Val Ile Asn Ser
   305                 310                 315                     320
   Leu Asn His Cys Leu Tyr Val Gly Cys Ala Ile Leu Gln Ser Leu Glu
                   325                 330                 335
   Ser Arg Asn Ile Pro Val Thr Trp Asn Leu Phe Ser Thr Phe Asp Met
               340                 345                 350
   Met Thr Tyr Gly Asp Asp Gly Val Tyr Met Phe Pro Met Met Phe Ala
           355                 360                 365
   Ser Val Ser Asp Gln Ile Phe Ala Asn Leu Thr Ala Tyr Gly Leu Lys
       370                 375                 380
   Pro Thr Arg Val Asp Lys Ser Val Gly Ala Ile Glu Pro Ile Asp Pro
   385                 390                 395                     400
   Glu Ser Val Val Phe Leu Lys Arg Thr Ile Thr Arg Thr Pro His Gly
                   405                 410                 415
   Ile Arg Gly Leu Leu Asp Arg Gly Ser Ile Ile Arg Gln Phe Tyr Tyr
               420                 425                 430
   Ile Lys Gly Glu Asn Ser Asp Asp Trp Lys Thr Pro Pro Lys Thr Ile
           435                 440                 445
   Asp Pro Thr Ser Arg Gly Gln Gln Leu Trp Asn Ala Cys Leu Tyr Ala
       450                 455                 460
   Ser Gln His Gly Pro Glu Phe Tyr Asn Lys Val Tyr Arg Leu Ala Glu
   465                 470                 475                 480
   Lys Ala Val Glu Tyr Glu Glu Leu His Phe Glu Pro Pro Ser Tyr His
                   485                 490                 495
   Ser Ala Leu Glu His Tyr Asn Asn Gln Phe Asn Gly Val Asp Thr Arg
               500                 505                 510
   Ser Asp Gln Ile Asp Ala Ser Val Met Thr Asp Leu His Cys Asp Val
           515                 520                 525
   Phe Glu Val Leu Glu
       530
<210> 4
   <211> 526
   <212> PRT
   <213> Artificial
<220>
   <223> Norovirus-RdRp having C-terminal his tag
<400> 4
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
   260 265 270
   Ala Gln Val Val Ala Glu Asp Leu Leu Ser Pro Ser Val Val Asp Val
   275 280 285
   Gly Asp Phe Lys Ile Ser Ile Asn Glu Gly Leu Pro Ser Gly Val Pro
   290 295 300
   Cys Thr Ser Gln Trp Asn Ser Ile Ala His Trp Leu Leu Thr Leu Cys
   305 310 315 320
   Ala Leu Ser Glu Val Thr Asn Leu Ser Pro Asp Ile Ile Gln Ala Asn
   325 330 335
   Ser Leu Phe Ser Phe Tyr Gly Asp Asp Glu Ile Val Ser Thr Asp Ile
   340 345 350
   Lys Leu Asp Pro Glu Lys Leu Thr Ala Lys Leu Lys Glu Tyr Gly Leu
   355 360 365
   Lys Pro Thr Arg Pro Asp Lys Thr Glu Gly Pro Leu Val Ile Ser Glu
   370 375 380
   Asp Leu Asn Gly Leu Thr Phe Leu Arg Arg Thr Val Thr Arg Asp Pro
   385 390 395 400
   Ala Gly Trp Phe Gly Lys Leu Glu Gln Ser Ser Ile Leu Arg Gln Met
   405 410 415
   Tyr Trp Thr Arg Gly Pro Asn His Glu Asp Pro Ser Glu Thr Met Ile
   420 425 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu His His His His His His
           515                 520                 525
<210> 5
   <211> 456
   <212> PRT
   <213> Artificial
<220>
   <223> Norovirus-RdRp having N-terminal His-tag
<400> 5
   Met His His His His His His Gly Gly Asp Ser Lys Gly Thr Tyr Cys
   1 5 10 15
   Gly Ala Pro Ile Leu Gly Pro Gly Ser Ala Pro Lys Leu Ser Thr Lys
   20 25 30
   Thr Lys Phe Trp Arg Ser Ser Thr Thr Pro Leu Pro Pro Gly Thr Tyr
   35 40 45
   Glu Pro Ala Tyr Leu Gly Gly Lys Asp Pro Arg Val Lys Gly Gly Pro
   50 55 60
   Ser Leu Gln Gln Val Met Arg Asp Gln Leu Lys Pro Phe Thr Glu Pro
   65 70 75 80
   Arg Gly Lys Pro Pro Lys Pro Ser Val Leu Glu Ala Ala Lys Lys Thr
   85 90 95
   Ile Ile Asn Val Leu Glu Gln Thr Ile Asp Pro Pro Glu Lys Trp Ser
   100 105 110
   Phe Thr Gln Ala Cys Ala Ser Leu Asp Lys Thr Thr Ser Ser Gly His
   115 120 125
   Pro His His Met Arg Lys Asn Asp Cys Trp Asn Gly Glu Ser Phe Thr
   130 135 140
   Gly Lys Leu Ala Asp Gln Ala Ser Lys Ala Asn Leu Met Phe Glu Gly
   145 150 155 160
   Gly Lys Asn Met Thr Pro Val Tyr Thr Gly Ala Leu Lys Asp Glu Leu
   165 170 175
   Val Lys Thr Asp Lys Ile Tyr Gly Lys Ile Lys Lys Arg Leu Leu Trp
   180 185 190
   Gly Ser Asp Leu Ala Thr Met Ile Arg Cys Ala Arg Ala Phe Gly Gly
   195 200 205
   Leu Met Asp Glu Leu Lys Ala His Cys Val Thr Leu Pro Ile Arg Val
   210 215 220
   Gly Met Asn Met Asn Glu Asp Gly Pro Ile Ile Phe Glu Arg His Ser
   225 230 235 240
   Arg Tyr Lys Tyr His Tyr Asp Ala Asp Tyr Ser Arg Trp Asp Ser Thr
   245 250 255
   Gln Gln Arg Ala Val Leu Ala Ala Ala Leu Glu Ile Met Val Lys Phe
   260 265 270
   Ser Ser Glu Pro His Leu Ala Gln Val Val Ala Glu Asp Leu Leu Ser
   275 280 285
   Pro Ser Val Val Asp Val Gly Asp Phe Lys Ile Ser Ile Asn Glu Gly
   290 295 300
   Leu Pro Ser Gly Val Pro Cys Thr Ser Gln Trp Asn Ser Ile Ala His
   305 310 315 320
   Trp Leu Leu Thr Leu Cys Ala Leu Ser Glu Val Thr Asn Leu Ser Pro
   325 330 335
   Asp Ile Ile Gln Ala Asn Ser Leu Phe Ser Phe Tyr Gly Asp Asp Glu
   340 345 350
   Ile Val Ser Thr Asp Ile Lys Leu Asp Pro Glu Lys Leu Thr Ala Lys
   355 360 365
   Leu Lys Glu Tyr Gly Leu Lys Pro Thr Arg Pro Asp Lys Thr Glu Gly
   370 375 380
   Pro Leu Val Ile Ser Glu Asp Leu Asn Gly Leu Thr Phe Leu Arg Arg
   385 390 395 400
   Thr Val Thr Arg Asp Pro Ala Gly Trp Phe Gly Lys Leu Glu Gln Ser
   405 410 415
   Ser Ile Leu Arg Gln Met Tyr Trp Thr Arg Gly Pro Asn His Glu Asp
   420 425 430
   Pro Ser Glu Thr Met Ile Pro His Ser Gln Arg Pro Ile Gln Leu Met
   435 440 445
   Ser Leu Leu Gly Glu Ala Ala Leu
   450 455
<210> 6
   <211> 526
   <212> PRT
   <213> Artificial
<220>
   <223> Sapovirus-RdRp having C-terminal His-tag
<400> 6
   Met Gly Gly Asp Ser Lys Gly Thr Tyr Cys Gly Ala Pro Ile Leu Gly
   1               5                   10                  15
   Pro Gly Ser Ala Pro Lys Leu Ser Thr Lys Thr Lys Phe Trp Arg Ser
               20                  25                  30
   Ser Thr Thr Pro Leu Pro Pro Gly Thr Tyr Glu Pro Ala Tyr Leu Gly
           35                  40                  45
   Gly Lys Asp Pro Arg Val Lys Gly Gly Pro Ser Leu Gln Gln Val Met
       50                  55                  60
   Arg Asp Gln Leu Lys Pro Phe Thr Glu Pro Arg Gly Lys Pro Pro Lys
   65                  70                  75                  80
   Pro Ser Val Leu Glu Ala Ala Lys Lys Thr Ile Ile Asn Val Leu Glu
                   85                  90                  95
   Gln Thr Ile Asp Pro Pro Glu Lys Trp Ser Phe Thr Gln Ala Cys Ala
               100                 105                 110
   Ser Leu Asp Lys Thr Thr Ser Ser Gly His Pro His His Met Arg Lys
           115                 120                 125
   Asn Asp Cys Trp Asn Gly Glu Ser Phe Thr Gly Lys Leu Ala Asp Gln
       130                 135                 140
   Ala Ser Lys Ala Asn Leu Met Phe Glu Gly Gly Lys Asn Met Thr Pro
   145                 150                 155                 160
   Val Tyr Thr Gly Ala Leu Lys Asp Glu Leu Val Lys Thr Asp Lys Ile
                   165                 170                 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
   260 265 270
   Ala Gln Val Val Ala Glu Asp Leu Leu Ser Pro Ser Val Val Asp Val
   275 280 285
   Gly Asp Phe Lys Ile Ser Ile Asn Glu Gly Leu Pro Ser Gly Val Pro
   290 295 300
   Cys Thr Ser Gln Trp Asn Ser Ile Ala His Trp Leu Leu Thr Leu Cys
   305 310 315 320
   Ala Leu Ser Glu Val Thr Asn Leu Ser Pro Asp Ile Ile Gln Ala Asn
   325 330 335
   Ser Leu Phe Ser Phe Tyr Gly Asp Asp Glu Ile Val Ser Thr Asp Ile
   340 345 350
   Lys Leu Asp Pro Glu Lys Leu Thr Ala Lys Leu Lys Glu Tyr Gly Leu
   355 360 365
   Lys Pro Thr Arg Pro Asp Lys Thr Glu Gly Pro Leu Val Ile Ser Glu
   370 375 380
   Asp Leu Asn Gly Leu Thr Phe Leu Arg Arg Thr Val Thr Arg Asp Pro
   385 390 395 400
   Ala Gly Trp Phe Gly Lys Leu Glu Gln Ser Ser Ile Leu Arg Gln Met
   405 410 415
   Tyr Trp Thr Arg Gly Pro Asn His Glu Asp Pro Ser Glu Thr Met Ile
   420 425 430
   Pro His Ser Gln Arg Pro Ile Gln Leu Met Ser Leu Leu Gly Glu Ala
   435 440 445
   Ala Leu His Gly Pro Ala Phe Tyr Ser Lys Ile Ser Lys Leu Val Ile
   450 455 460
   Ala Glu Leu Lys Glu Gly Gly Met Asp Phe Tyr Val Pro Arg Gln Glu
   465 470 475 480
   Pro Met Phe Arg Trp Met Arg Phe Ser Asp Leu Ser Thr Trp Glu Gly
   485 490 495
   Asp Arg Asn Leu Ala Pro Ser Phe Val Asn Glu Asp Gly Val Glu Val
   500 505 510
   Asp Lys Leu Ala Ala Ala Leu Glu His His His His His His
   515 520 525
<210> 7
   <211> 523
   <212> PRT
   <213> Artificial
<220>
   <223> Sapovirus-RdRp having N-terminal His-tag
<400> 7
   Met Lys His His His His His His Asp Glu Phe Gln Trp Lys Gly Leu
   1               5                   10                  15
   Pro Val Val Lys Ser Gly Leu Asp Val Gly Gly Met Pro Thr Gly Thr
               20                  25                  30
   Arg Tyr His Arg Ser Pro Ala Trp Pro Glu Glu Gln Pro Gly Glu Thr
           35                  40                  45
   His Ala Pro Ala Pro Phe Gly Ala Gly Asp Lys Arg Tyr Thr Phe Ser
       50                  55                  60
   Gln Thr Glu Met Leu Val Asn Gly Leu Lys Pro Tyr Thr Glu Pro Thr
   65                  70                  75                  80
   Ala Gly Val Pro Pro Gln Leu Leu Ser Arg Ala Val Thr His Val Arg
                   85                  90                  95
   Ser Tyr Ile Glu Thr Ile Ile Gly Thr His Arg Ser Pro Val Leu Thr
               100                 105                 110
   Tyr His Gln Ala Cys Glu Leu Leu Glu Arg Thr Thr Ser Cys Gly Pro
           115                 120                 125
   Phe Val Gln Gly Leu Lys Gly Asp Tyr Trp Asp Glu Glu Gln Gln Gln
       130                 135                 140
   Tyr Thr Gly Val Leu Ala Asn His Leu Glu Gln Ala Trp Asp Lys Ala
   145                 150                 155                 160
   Asn Lys Gly Ile Ala Pro Arg Asn Ala Tyr Lys Leu Ala Leu Lys Asp
                   165                 170                 175
   Glu Leu Arg Pro Ile Glu Lys Asn Lys Ala Gly Lys Arg Arg Leu Leu
               180                 185                 190
   Trp Gly Cys Asp Ala Ala Thr Thr Leu Ile Ala Thr Ala Ala Phe Lys
           195                 200                 205
   Ala Val Ala Thr Arg Leu Gln Val Val Thr Pro Met Thr Pro Val Ala
       210                 215                 220
   Val Gly Ile Asn Met Asp Ser Val Gln Met Gln Val Met Asn Asp Ser
   225                 230                 235                 240
   Leu Lys Gly Gly Val Leu Tyr Cys Leu Asp Tyr Ser Lys Trp Asp Ser
                   245                 250                 255
   Thr Gln Asn Pro Ala Val Thr Ala Ala Ser Leu Ala Ile Leu Glu Arg
               260                 265                 270
   Phe Ala Glu Pro His Pro Ile Val Ser Cys Ala Ile Glu Ala Leu Ser
           275                 280                 285
   Ser Pro Ala Glu Gly Tyr Val Asn Asp Ile Lys Phe Val Thr Arg Gly
       290                 295                 300
   Gly Leu Pro Ser Gly Met Pro Phe Thr Ser Val Val Asn Ser Ile Asn
   305                 310                 315                 320
   His Met Ile Tyr Val Ala Ala Ala Ile Leu Gln Ala Tyr Glu Ser His
                   325                 330                 335
   Asn Val Pro Tyr Thr Gly Asn Val Phe Gln Val Glu Thr Val His Thr
               340                 345                 350
   Tyr Gly Asp Asp Cys Met Tyr Ser Val Cys Pro Ala Thr Ala Ser Ile
           355                 360                 365
   Phe His Ala Val Leu Ala Asn Leu Thr Ser Tyr Gly Leu Lys Pro Thr
       370                 375                 380
   Ala Ala Asp Lys Ser Asp Ala Ile Lys Pro Thr Asn Thr Pro Val Phe
   385                 390                 395                 400
   Leu Lys Arg Thr Phe Thr Gln Thr Pro His Gly Val Arg Ala Leu Leu
                   405                 410                 415
   Asp Ile Thr Ser Ile Thr Arg Gln Phe Tyr Trp Leu Lys Ala Asn Arg
               420                 425                 430
   Thr Ser Asp Pro Ser Ser Pro Pro Ala Phe Asp Arg Gln Ala Arg Ser
           435                 440                 445
   Ala Gln Leu Glu Asn Ala Leu Ala Tyr Ala Ser Gln His Gly Pro Val
       450                 455                 460
   Val Phe Asp Thr Val Arg Gln Ile Ala Ile Lys Thr Ala Gln Gly Glu
   465                 470                 475                 480
   Gly Leu Val Leu Val Asn Thr Asn Tyr Asp Gln Ala Leu Ala Thr Tyr
                   485                 490                 495
   Asn Ala Trp Phe Ile Gly Gly Thr Val Pro Asp Pro Val Gly His Thr
               500                 505                 510
   Glu Gly Thr His Lys Ile Val Phe Glu Met Glu
           515                 520
<210> 8
   <211> 539
   <212> PRT
   <213> Artificial
<220>
   <223> Vesivirus-RdRp having C-terminal His-tag
<400> 8
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145 150 155 160
   Ala Thr Gln Gly Ile Gly Leu Pro His Glu Tyr Thr Ile Gly Leu Lys
   165 170 175
   Asp Glu Leu Arg Pro Val Glu Lys Val Gln Glu Gly Lys Arg Arg Met
   180 185 190
   Ile Trp Gly Cys Asp Val Gly Val Ala Thr Val Cys Ala Ala Ala Phe
   195 200 205
   Lys Gly Val Ser Asp Ala Ile Thr Ala Asn His Gln Tyr Gly Pro Val
   210 215 220
   Gln Val Gly Ile Asn Met Asp Gly Pro Ser Val Glu Ala Leu Tyr Gln
   225 230 235 240
   Arg Ile Arg Ser Ala Ala Lys Val Phe Ala Val Asp Tyr Ser Lys Trp
   245 250 255
   Asp Ser Thr Gln Ser Pro Arg Val Ser Ala Ala Ser Ile Asp Ile Leu
   260 265 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
           435                 440                 445
   Asp Pro Thr Ser Arg Gly Gln Gln Leu Trp Asn Ala Cys Leu Tyr Ala
       450                 455                 460
   Ser Gln His Gly Pro Glu Phe Tyr Asn Lys Val Tyr Arg Leu Ala Glu
   465                 470                 475                 480
   Lys Ala Val Glu Tyr Glu Glu Leu His Phe Glu Pro Pro Ser Tyr His
                   485                 490                 495
   Ser Ala Leu Glu His Tyr Asn Asn Gln Phe Asn Gly Val Asp Thr Arg
               500                 505                 510
   Ser Asp Gln Ile Asp Ala Ser Val Met Thr Asp Leu His Cys Asp Val
           515                 520                 525
   Phe Glu Val Leu Glu His His His His His His
       530                 535
<210> 9
   <211> 22
   <212> RNA
   <213> Homo sapiens
<400> 9
   uuuguucguu cggcucgcgu ga 22
<210> 10
   <211> 25
   <212> RNA
   <213> Artificial
<220>
   <223> miRNA375 with modified sequence
<400> 10
   uuuguucguu cggcucgcgu gaccc 25

## Description

The present invention relates to a kit for carrying out methods for the detection of target RNA sequences making use of strand displacement techniques employing RNA-dependent RNA polymerases of viruses of the *Caliciviridae* family having RNA-oligonucleotide duplex separation activity and being capable of *de novo* RNA synthesis in the absence of a primer.

The concept of detecting nucleic acid sequences using strand displacement and its employment in PCR amplification reactions has been introduced by Gelfand et al. in the early 1990s (see WO-A-1992/002638). Corresponding methods and kits are commercially available from Roche Diagnostics and are known under the TaqMan technology. The TaqMan principle particularly employs the 5' to 3'-nuclease activity of nucleic acid (in particular DNA) polymerases.

While the TaqMan technology is mainly directed to DNA constructs and, thus, makes use of DNA-dependent DNA polymerases, further studies have shown that also RNA-dependent RNA polymerases have the ability to displace pre-hybridised nucleotide sequences from a template strand when processing from regions downstream to the preformed RNA duplex. For example, Cho et al. (1993), Journal of Virology 67 (6), pages 3010-3018, show that poliovirus RNA-dependent RNA polymerase (RdRp) shows such an RNA duplex separation activity and is capable of displacing a 1000 nt antisense sequence from the template strand. The poliovirus RdRp is, however, a primer-dependent enzyme, i.e. for polymerization of a complementary RNA strand to occur an oligonucleotide primer is required.

Thus, the existing techniques especially for RNA detection and RNA amplification making use of strand displacement have several limitations: the TaqMan approach needs an enzyme having proof reading activity, i. e. 5' to 3'-nuclease activity. Furthermore, at least two oligonucleotides are required: (1) a labelled oligonucleotide for pre-hybridisation to the target sequence and (2) at least one primer for priming the polymerase activity. The latter drawback is also valid for the study according to Cho et al., *supra,* since the poliovirus RdRp is a primer-dependent enzyme as well. The poliovirus RdRp is also capable of initiation of RNA synthesis by back-priming that occurs after elongation of the 3'-terminus by the RdRp and snapping back of the elongated tail on the template leading through priming to initiation of RNA synthesis. The poliovirus RdRp is not capable of initiation *de novo,* i.e. without priming. The present invention is directed to a kit for detecting a target RNA sequence in a sample comprising:
- at least one labelled oligonucleotide containing a sequence complementary to a region of the target RNA;
- an RNA-dependent RNA polymerase (RdRp) of a virus of the *Caliciviridae* family having an RNA duplex separation activity under polymerisation conditions and being capable of *de novo* RNA synthesis in the absence of a primer;
wherein the at least one oligonucleotide is blocked at its 3'-end.

As used herein, a "sample" refers to any substance containing or presumed to contain RNA and includes a sample of tissue or fluid isolated from an individual or individuals, including but not limited, for example, skin, plasma, serum, spinal fluid, lymph fluid, synovial fluid, urine, tears, blood cells, organs, tumors, and also to samples of *in vitro* cell culture constituents (including, but not limited to, conditioned medium resulting from the growth of cells in cell culture medium, recombinant cells and cell components). The "labelled" oligonucleotide to be used in the methods claimed in the present invention is not necessarily physically derived from any existing or natural sequence but may be generated in any manner, including chemical synthesis, DNA replication, reverse transcription or a combination thereof.

The oligonucleotide according to the present invention is blocked at its 3'-end and a single-stranded nucleic acid and maybe DNA or RNA. The "oligonucleotide" according to the present invention may also be denoted as "oligoprobe" and may be a polynucleotide of any length. RNA oligonucleotides, i.e. oligriboonucleotides are preferred. The term "oligonucleotide" furthermore intends a polynucleotide of genomic DNA or RNA, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature; and (3) ins not found in nature.

The "oligonucleotide" is further preferred to be a rather small polynucleotide, i.e. preferably it has a length of from 5 to 100, more preferred 5 to 20, most preferred 10 to 12 nucleotides.

The term "label" as used herein refers to any atom or molecule which can be used to provide a detectable (preferably quantifiable) signal, and which can be attached to a nucleic acid, in particular the oligonucleotide. Labels may provide signals detectable by fluorescence, radioactivity, colorimetry, gravimetry, X-ray diffraction or absorption, magnetism, enzymatic activity, and the like. Preferred labels according to the present invention are fluorescent labels.

The single-stranded RNA in the sample may as well be of any origin and length. Typical examples of single-stranded RNA species to be detected are messenger RNA, viral RNA and the like. Since the present invention is particular applicable to situations in which smaller RNA species are detected, the single-stranded RNA has preferably a length of from 18 to 200, preferably 16 to 40, most preferably 10 to 25 nucleotides.

Further preferred examples of target RNAs that may be detected by using the kit according to the invention are microRNA species (also denoted as "miRNA" or "miR"). miRNAs have a role in gene regulation through translational silencing and are encoded by specific genes. Especially in humans, miRNA disruption has been described in association with several cancer forms. Therefore, miRNA profiling has important implications for cancer aetiology, diagnosis and treatment (see, e.g. Esquela-Kerscher et al. (2006) Nat. Rev. Cancer 6, 259-269; Calin et al. (2006) Nat Rev. Cancer 6, 857-866). Using the kit of the invention, it would, for example, be possible to detect specific miRNAs (or disrupted miRNAs) using labelled oligonucleotides specific for such targets.

In particular with respect to detection of RNA species of shorter length, as exemplified above, it is desirable that the single-stranded RNA contains at least one C at its 3' end, more preferably having an (C)ₙ 3'-terminal repeat (also denoted as "polyC" repeat) with n being integer of at least 2 or 3.

Crucial to the present invention is the activity of special RNA-dependent RNA polymerases of viruses of the *Caliciviridae* family capable of displacing oligonucleotides annealed to the target RNA and being furthermore capable of *de novo* RNA synthesis in the absence of any primer.

Enzymes of this category typically have the feature that *de novo* RNA synthesis can be accomplished on an ssRNA strand. Such RNA-dependent RNA polymerases typically show a "right hand conformation" and have a primary sequence comprising a conserved arrangement of the following sequence motives:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid.

The so-called "right hand conformation as used herein means that the tertiary structure (conformation) of the protein folds like a right hand with finger, palm and thumb, as observed in most template-dependent polymerases.

The sequence motif "XXDYS" is the so-called A-motif. The A-motif is responsible for the discrimination between ribonucleosides and deoxyribonucleosides. The motif "GXPSG" is the so-called B-motif. The B-motif is conserved within all representatives of the RdRp family of the corresponding polymerases from *Calicivirdae.* The motif "YGDD" ("C-motif') represents the active site of the enzyme. This motif, in particular the first aspartate residue (in bold, YGDD) plays an important role in the coordination of the metal ions during the Mg²⁺/Mn²⁺-dependent catalysis. The motif "XXYGL" is the so-called D-motif. The D-motif is a feature of template-dependent polymerases. Finally, the "XXXXFLXRXX" motif ("E-motif") is a feature of RNA-dependent RNA polymerases which discriminates them from DNA-dependent RNA polymerases.

The RdRps of the calicivirus family are capable of synthesizing complementary strands using as a template any ssRNA template *in vitro,* including heterologous viral, eukaryotic and prokaryotic templates. The ssRNA template may be positive stranded or negative stranded. The RdRp for use in the present invention is capable of synthesizing a complementary strand to the ssRNA to be detected by *de novo* synthesis in the absence of a primer.

The term *"de novo* synthesis in the absence of a primer" in the context of the present invention means that the RdRp is capable of synthesising a complementary RNA strand on a single-stranded RNA template without requiring an RNA duplex (either formed by a separate primer molecule or by back folding of the template) for initiation of polymerisation. For *de novo* RNA synthesis to occur, the RNA target/template should not contain a polyU, polyA or polyG sequence at its 3'-end. Preferably, such *de novo* synthesis occurs on a ssRNA template having at least one C at its 3' end, more preferably having an (C)ₙ 3'-terminal repeat with n being integer of at least 2 or 3.

Especially in cases of detecting RNA having a (C)n 3'-terminal repeat, it is preferred that GTP is added in surplus (preferably, 2x 3x, 4x or 5x more) over ATP, UTP and CTP, respectively, in step (b) or (ii) respectively, as defined above.

Preferred embodiments of the RdRp are corresponding enzymes of a human and/or non-human pathogenic calicivirus. Especially preferred is an RdRp of a norovirus, sapovirus, vesivirus or lagovirus, for example the RdRp of the norovirus strain HuCV/NL/Dresden174/1997/GE (GenBank Acc. No. AY741811) or of the sapovirus strain pJG-Sap01 (GenBank Acc. No. AY694184) or an RdRp of the vesivirus strain FCV/Dresden/2006/GE (GenBank Acc. No. DQ424892).

According to especially preferred embodiments of the invention the RdRp is a protein having an amino acid sequence according SEQ ID NO: 1 (norovirus-RdRp), SEQ ID NO: 2 (sapovirus-RdRp) or SEQ ID NO: 3 (vesivirus-RdRp). The person skilled in the art is readily capable of preparing such RdRp, for example by recombinant expression using suitable expression vectors and host organisms (cf. WO-A-2007/012329). To facilitate purification of the RdRp in recombinant expression, it is preferred that the RdRp is expressed with a suitable "tag" (for example GST or (His)₆-tag) at the N- or C-terminus of the corresponding sequence. For example, a histidine tag allows the purification of the protein by affinity chromatography over a nickel or cobalt column in a known fashion. Examples of embodiments of RdRps fused to a histidine tag are the proteins having an amino acid sequence according to SEQ ID NO: 4, SEQ ID NO: 5, SEQ ID NO: 6 or SEQ ID NO: 7. SEQ ID NO: 4 and SEQ ID NO: 5 correspond to a norovirus-RdRp having a histidine tag (SEQ ID NO: 4: C-terminal His-tag; SEQ ID NO: 5: N-terminal His-tag). SEQ ID NO: 6 and SEQ ID NO: 7 correspond to the amino acid sequence of a sapovirus-RdRp having a histidine tag (SEQ ID NO: 6: C-terminal His-tag; SEQ ID NO: 7: N-terminal His-tag). SEQ ID NO: 8 corresponds to the amino acid sequence of vesirius-RdRp having a histidine tag (C-terminal).
SEQ ID NO: 1: SEQ ID NO: 2: SEQ ID NO: 3: SEQ ID NO: 4: SEQ ID NO: 5: SEQ ID NO: 6: SEQ ID NO: 7: SEQ ID NO: 8:

Another characteristic feature of RdRps from *Caliciviridae,* is a terminal transferase activity. With respect to the terminal transferase activity of the enzymes according to the present invention, see Rohayem et al. (2006) Journal of General Virology 87, 2621-2630, and the reaction conditions for accessing terminal transferase activity of calicivirus RdRps described therein.

The labelled oligonucleotide used herein is selected to be "substantially" complementary to a region of the target RNA (i. e. the RNA sequence to be detected). Thus, the oligonucleotide needs not to reflect the exact sequence of the target, but must be sufficiently complementary to at least a region of the target for hybridising selectively to it. Non-complementary bases or longer sequences can be interspersed into the oligonucleotide or located at the ends of the oligonucleotide, provided that it retains sufficient complementarity with the template strand to form a stable duplex therewith.

The oligonucleotides may be prepared by any suitable methods. Methods for preparing oligonucleotides of specific sequence are known in the art, and include, for example, cloning and restriction of appropriate sequences and direct chemical synthesis. Chemical synthesis methods may include, for example, the phosphotriester method described by Narang et al. (1979) Method in Enzymology 68:90, the phoshpdiester method disclosed by Brown et al. (1979) Methods in Enzymology 68:109, the diethylphosphoramidate method disclosed in Beaucage et al. (1981) Tetrahedron Letters 22:1859, and the solid support method disclosed in US-A-4,458,066.

The oligonucleotide is labelled, as described below, by incorporating moieties detectable by spectroscopic, photochemical, biochemical, immunochemical, or chemical means. The method of linking or conjugating the label to the oligonucleotide depends, of course, on the type of label(s) used and the position of the label on the oligonucleotide.

A variety of labels that would be appropriate for use in the present invention, as well as methods for their inclusion in the oligonucleotide, are known in the art and include, but are not limited to, enzymes (for example alkaline phosphatase and horseradish peroxidase) and enzyme substrates, radioactive atoms, fluorescent dyes, chromophores, chemiluminescent labels, electrochemiluminescent labels, such as Origen™ (Igen), ligands having specific binding partners or any other labels that may interact with each other to enhance, alter, or diminish a signal.

Among radioactive atoms, ³²P is preferred. Methods for introducing ³²P into nucleic acids are known in the art, and include, for example 5'-labeling with a kinase, or random insertion by nick translation. Enzymes are typically detected by their activity. "Specific binding partner" refers to a protein capable of binding a ligand molecule with high specificity, as for example in the case of an antigen and a monoclonal antibody specific therefore. Other specific binding partners include biotin and avidin or streptavidin, IgG and protein A, and the numerous receptor-ligand couples known in the art. The above description is not meant to categorize the various labels into distinct classes, as the same label may serve in several different modes. For example ¹²⁵I may serve as a radioactive label or as an electron-dense reagent. HRP may serve as enzyme or as antigen for a monclonal antibody. Further, one may combine various labels for a desired effect. For example, one might label a probe with biotin, and detect the presence of the oligonucleotide with avidin labelled with ¹²⁵I, or with an anti-biotin monoclonal antibody labelled with HRP. Other permutations and possibilities will be readily apparent for the skilled person and considered as equivalents within the scope of the present invention.

Fluorophores for use as labels in constructing the labelled oligonucleotide according to the present invention are preferred and include rhodamine and derivatives, such as Texas Red, 5-carboxytetramethyl rhodamine (5-TAMRA), 6-carboxytetramethyl rhodamine (6-TAMRA) and their respective succinimidyl esters, fluorescein and derivatives, such as 5-bromomethyl fluorescein, 5-carboxy fluorescein (5-FAM), 6-carboxy fluorescein (6-FAM), and their respective succinimidyl esters, Lucifer Yellow, IAEDANS, 7-Me₂-N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromotrimethyl-ammoniobimane.

It is further preferred, that, if fluorescence is used to detect the released oligonucleotide, the ssRNA is provided with a molecule quenching the fluorescence of the fluorescent label of the oligonucleotide when it is hybridised to the target RNA. For example, the oligonucleotide may be labelled with a fluorescein derivative, such as 5- or 6-FAM, preferably at the 5'-end thereof, and the ssRNA is provided with a quencher for the fluorescein label, for example 5-TAMRA or 6-TAMRA.

Furthermore, in this situation it is also preferred that the oligonucleotide comprising the (fluorescein) label, preferably at its 5'-end, hybridises to the target sequence near its 5'-end which carries the quencher molecule. The quencher/donor may be generally selected as FRET (fluorescence resonance energy transfer) pairs such as in the case of FAM/TAMRA. However, it may also be desirable to select a so-called "dark quencher" (such as Dabcyl, methyl orange).

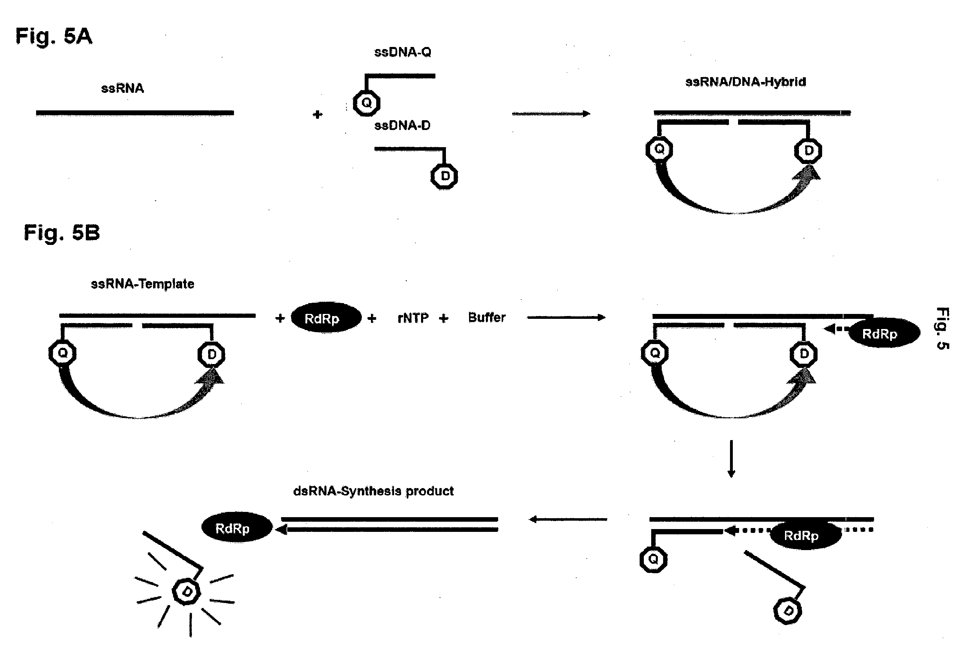

According to a further embodiment of the present invention the fluorescence donor-quencher pair can be provided in the form of two oligonucleotides (which may each be of RNA or DNA type) substantially complementary (with respect to the meaning of "substantially complementary" it is referred to the corresponding section above) to the target RNA but hybridising thereto at different, non-overlapping regions of said target RNA, and whereby the regions of complementarity to the target RNA are selected such that, when the oligonucleotides are hybridized to the target RNA, the fluorescence by the labelled oligonucleotide is quenched by the second oligonucleotide carrying the quencher, in particular by FRET between the fluorescence donor and quencher. Thus, when both donor and quencher oligonucleotides are hybridized to the target RNA, no or only a minor fluorescence signal is detected. When the RdRp according to the present invention initiates *de novo* RNA synthesis and synthesises an RNA strand complementary to the target RNA, it displaces both the donor and the quencher oligonucleotide from the target, and fluorescence emitted by the donor can be detected (see Fig. 5). In the illustrative example of Fig. 5, the quencher oligo has the quenching moiety at its 5'-end and hybridizes to or near the 5'-end of the target RNA. The donor oligo has the fluorescence donor at its 3'-end and hybridizes to or near the 3'end of the target RNA. Of course, quencher and donor moieties can be present at either probe. Furthermore, a quencher moiety could be present at the 3'-end of one oligo hybridizing to or near the 5'-end of the target and the donor group could be bound to the 5'-end of the other oligo (i.e. the labelled oligonucleotide) hybridizing near or to the 3'-end of the target RNA, and vice versa. In the latter scenario, it would be possible to use hybridizing regions which are farther remote from one another. With regard to preferred lengths and other characteristics of the quencher oliconucleotide (i.e. the second oligonucleotide present as a further component of the inventive kit) it is referred to the above sections in connection with the labelled oligonucleotide (i.e. the donor oligo). Examples of useful donor/quencher pairs in the context of the present invention have already been described above. Particularly preferred examples for providing donor/quencher pairs in the form of two oligonucleotides are a 5- or 6-FAM labelled oligonucleotide (donor) and a 5- or 6-TAMRA-coupled oligonucleotide (quencher).

Further preferred embodiments of the invention make use of so-called Molecular Beacons as labelled oligonucleotides. Molecular Beacons are described in US-A-5,925,517.

In this context, it should be noted that "hybridizing at the 5'-end" or hybridizing at the 3'-end" does necessarily mean that the labelled oligonucleotide (or second oligonucleotide having a quenching moiety) hybridises exactly to the 5'-end or 3'-end, respectively, of the target sequence. Rather, these terms also include that the oligonucleotide may hybridize near the 5'-end or 3'-end, respectively, which means that there may be additional nucleotides within the target sequence between its very 5'-end or 3'-end, respectively, of the sequence hybridizing with the respective oligonucleotide.

The labels for use in the present invention may be attached to the oligonucleotide directly or indirectly by a variety of techniques. Depending on the precise type of label used, the label can be located at the 5'- or the 3'-end of the oligonucleotide, located internally in the oligonucleotide or attached to spacer arms of various sizes and compositions to facilitate signal interactions. Using commercially available phosphoramidite reagents, one can produce oligomers containing functional groups (e.g., thiols or primary amines) at either the 5'- or the 3'-terminus via an appropriately protected phosphoramidite, and can label them using protocols described in, for example, PCR Protocols: A Guide to Methods and Applications (Innis et al., eds. Academic Press, 1990). The same holds true for chemical quenching moieties for an optionally present quencher oligonucleotide.

Methods for introducing oligonucleotide functionalizing reagents to introduce one or more sulfhydryl, amino or hydroxyl moieties into the oligonucleotide, typically at the 5'-terminus, are described in US-A4,914,210. A 5'-phosphate group can be introduced as a radioisotope by using polynucleotide kinase and gamma-³²P-ATP to provide a reporter group. Biotin can be added to the 5'-end by reacting an aminothymidine residue, or a 6-amino hexyl residue, introduced during synthesis, with an N-hydroxysuccinimide ester of biotin. Labels at the 3'-terminus may employ polynucleotide terminal transferase to add the desired moiety, such as for example, cordycepin³⁵S-dATP, and biotinylated dUTP.

Oligonucleotide derivatives are also available labels. For example, etheno-dA and etheno-A are known fluorescent adenine nucleotides that can be incorporated into an oligonucleotide. Similarly, etheno-dC or 2-amino purine deoxyriboside is another analogue that could be used in oligonucleotide synthesis. The oligonucleotides containing such nucleotide derivatives may be hydrolyzed to release much more strongly fluorescent mononucleotides as the RdRp unwinds the duplex formed between the target ssRNA and the labelled oligonucleotide.

According to the present invention, the term "RNA polymerization conditions" means the conditions, in particular relating to buffer, temperature, salt and metal ion (if applicable), that allow the RdRp to synthesize an RNA strand complementary to a template strand in the absence of a primer. Appropriate buffer, salt, metal ion, reducing agent (if applicable) and other conditions of RdRps are known to the skilled person. With regard to RdRps of caliciviruses, it is referred to WO-A-2007/012329. Thus, in typical examples of the methods according to the present invention, the ssRNA template is used in amounts of e.g. 1 µg to 4 µg per 50 µl reaction volume. The concentration of the ribonucleoside triphosphates is preferably in the range of from 0.1 µmol/l to 1 µmol/l, for example 0.4 µmol/l. The concentration of the RdRp may be for example 1 µmol/l to 6 µmol/l.

Typical buffer conditions are 10 to 80 mM, more preferred 20 to 50 mM HEPES pH 8.0, 1 to 4 mM, for example 3 mM magnesium acetate, magnesium chloride, manganese acetate or manganese chloride and 1 to 4 mM of a reducing agent, for example DTT. With respect to the terminal transferase activity of the RdRp, substantially the same conditions apply except that only one ribonucleotide is present, e.g. rCTP for providing a C-terminal repeat.

The RNA detection method may be stopped by introducing a stop solution into the reaction mixture. A typical stop solution contains 2 to 10 mM, preferably 4 to 8 mM ammonium acetate and 50 to 200 mM, for example 100 mM EDTA.

The reagents applied in the methods according to the present invention can be packaged into diagnostic ktis. Diagnostic kits include the labelled oligonucleotide and the RdRp of a virus of the *Caliciviridae* family. The labelled oligonucleotide is blocked at its 3'-end and comprises a label as described above. The kit may further contain a second oligonucleotide as described above comprising a quenching moiety, preferably at its 5'- or 3'-end. If the quencher moiety is present at the 5'-end, the second oligonucleotide is preferably blocked at its 3'-end. The kit may also contain other suitably packaged reagents and materials needed for carrying out the methods according to the present invention, for example, buffers, ribonucleotides (rATP, rGTP, rCTP, rUTP, and, optionally, a stop solution (preferably a stop solution as defined above, more preferred in the form of a 5x or 10x stop solution) and, also optionally, a helicase as well as instructions for conducting the methods.

The figures show:

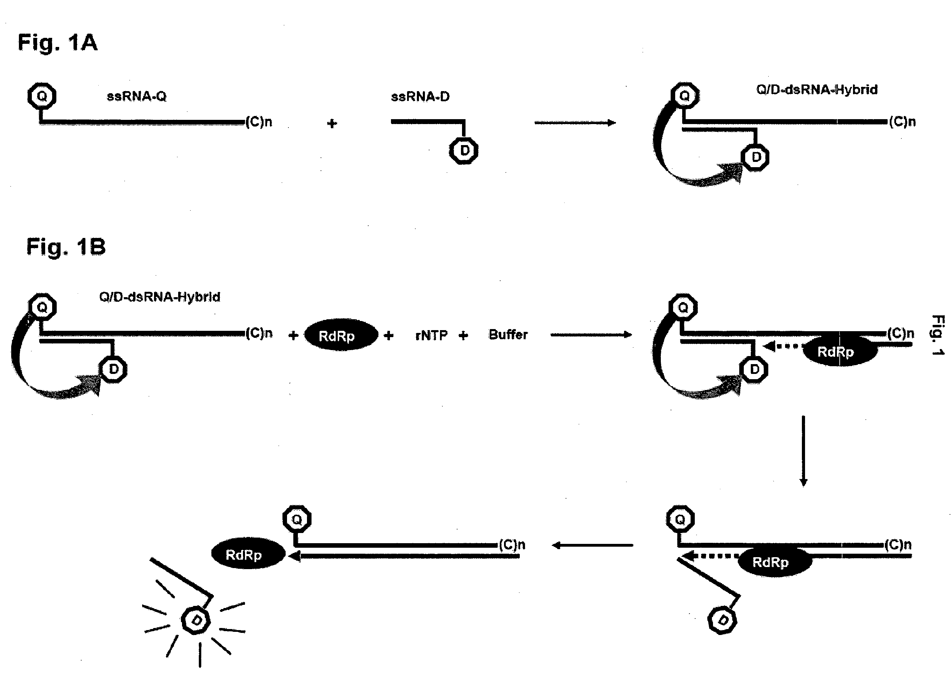

- Fig. 1: is a schematic illustration of a preferred embodiment of an RNA detection method showing the generation of a quencher/donor double-stranded RNA hybrid (Q/D-dsRNA-hybrid) used as a template. Fig. 1A: The hybrid is generated by hybridizing the single-stranded RNA olgionucleotide labelled at its 5'-terminus with the quencher (ssRNA-Q) and the single-stranded RNA labelled at its 5'-terminus with the donor (ssRNA-D). The ssRNA-Q and ssRNA-D bear complementary sequences. Emission of energy by the donor is attenuated by the quencher through resonance energy transfer. The hybridization reaction mixture contains equimolar concentrations of ssRNA-Q and ssRNA-D, incubated in the hybridization buffer at 65 °C for 30 min, then chilled on ice for 15 min. The hybridization buffer contains Tris-HCl 10 mM pH 8.0, NaCl 20 mM, EDTA 1 mM. Fig. 1B: schematic representation of strand displacement by the calicivirus RNA-dependent RNA polymerase (RdRp). The RdRp synthesizes RNA starting at the 3'-terminus of the template (ssRNA-Q), and displacing the ssRNA-D hybridized at the 5'-end of the ssRNA-Q. The ssRNA-Q template strand has a (C)ₙ nucleotide sequence at its 3'-terminus (with n ≥ 3). RNA synthesis results in a double-stranded RNA molecule consisting of the single-stranded RNA labelled at its 5'-terminus with the quencher (ssRNA-Q) and its complementary single-stranded RNA synthesized by the RdRp, as indicated. Strand displacement leads to release of the ssRNA-D from the ssRNA-Q, with subsequent detection of the energy emitted by the donor.

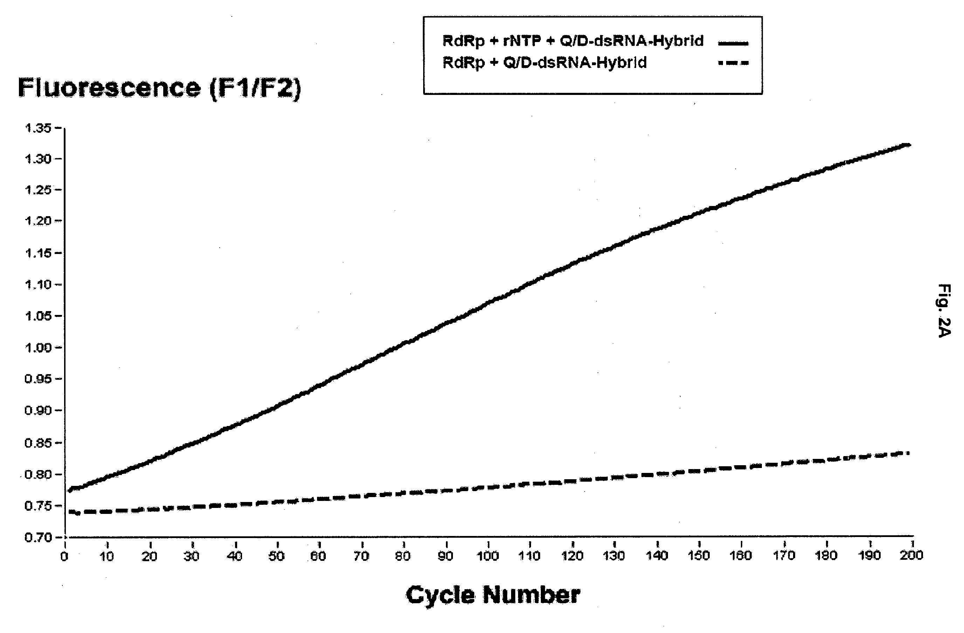

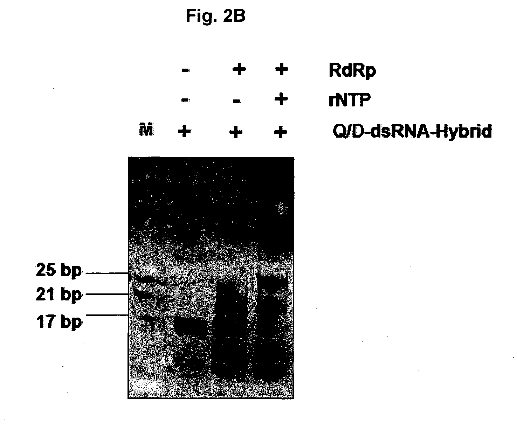

- Fig. 2: shows results of experiments demonstrating strand displacement of a labelled oligoribonucleotide from a template ssRNA strand by calicivirus RdRp. Fig. 2A: graphical representation of fluoresecence emission dependent on reaction cycle number. Filled line: reaction containing calicivirus RdRp, ribonucleotide triphosphates (rNTP), RdRp buffer, and Q/D-dsRNA hybrid. Dashed line: negative control reaction in the absence of rNTPs. Fig. 2B: photopgraph showing the products of the reactions of Fig. 2A analyzed using native polyacrylamide gel electrophoresis (PAGE). Lanes (from left to right): M: molecular size marker (base pairs (bp) are indicated); 1^{st} reaction lane: reaction containing Q/D-dsRNA hybrid only; 2^{nd} reaction lane: control reaction containing RdRp and Q/D-dsRNA hybrid, but no rNTPs; 3^{rd} reaction lane: reaction containing all required components (RdRp, rNTPs, Q/D-dsRNA hybrid).

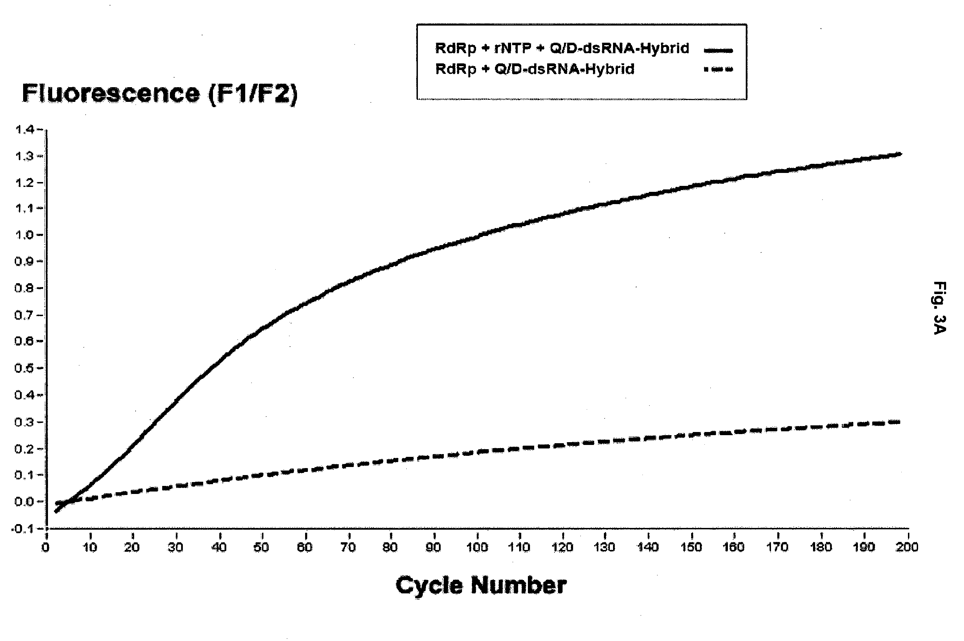

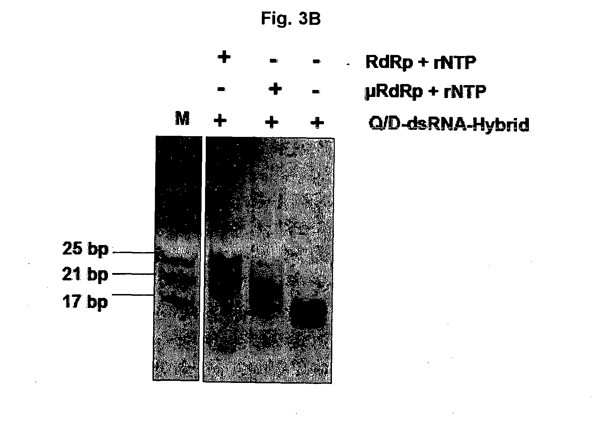

- Fig. 3: shows results of further experiments demonstrating strand displacement of a labelled oligoribonucleotide from a template ssRNA strand by calicivirus RdRp. Fig. 3A: graphical representation of fluorescence emission dependent on reaction cycle number. Filled line: reaction containing calicivirus RdRp, ribonucleotide triphosphates (rNTP), RdRp buffer, and Q/D-dsRNA hybrid. Dashed line: control reaction in the presence of an active site mutant of the RdRp (µRdRp) leading to total inhibition of RNA synthesis by the enzyme. Fig. 3B: photograph showing the products of the reactions of Fig. 3A analyzed using native polyacrylamide gel electrophoresis (PAGE). Lanes (from left to right): M: molecular size marker (base pairs (bp) are indicated); 1^{st} reaction lane: reaction containing calicivirus RdRp, rNTPs and Q/D-dsRNA hybrid; 2^{nd} reaction lane: control reaction containing µRdRp, rNTPs and Q/D-dsRNA hybrid; 3^{rd} reaction lane: control reaction containing Q/D-dsRNA hybrid only.

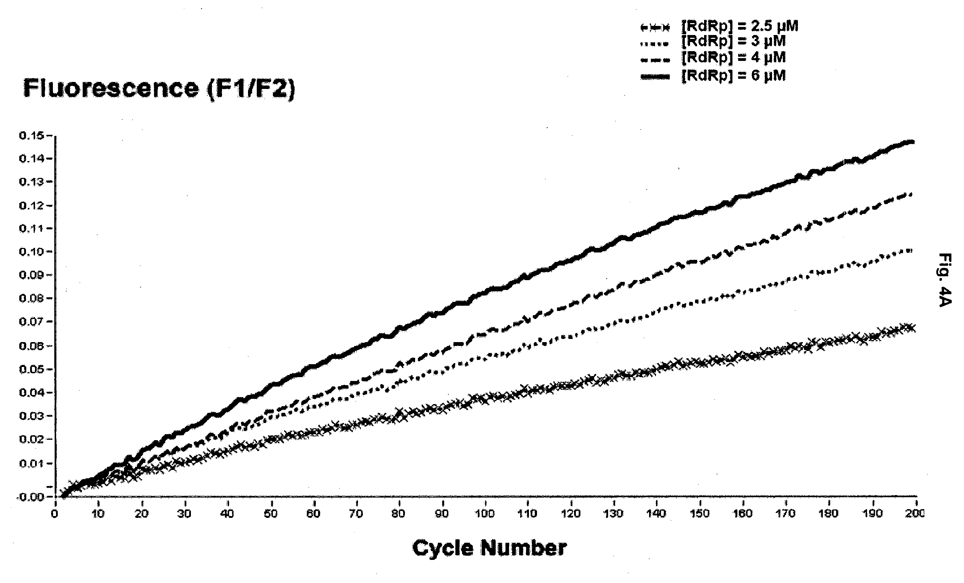

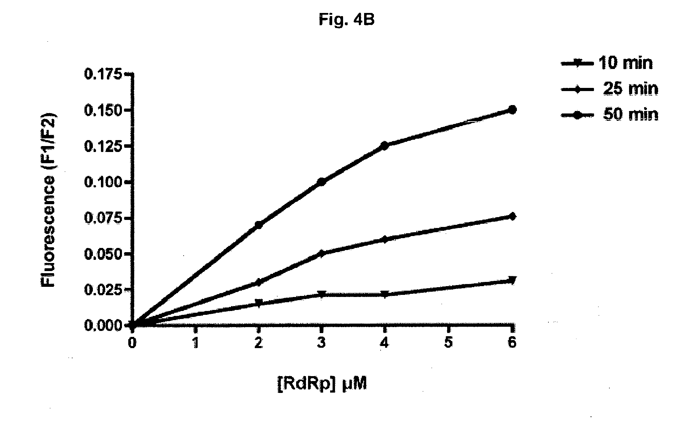

- Fig. 4: shows the results of experiments demonstrating the concentration- and time-dependent synthesis of dsRNA through strand displacement by the calicivirus RdRp. Fig. 4A: graphical representation of fluoresecence emission dependent on reaction cycle number. The reaction mix contained increasing concentrations of RdRp (2.5 µM, 3 µM, 4 µM, and 6 µM; as indicated). Fig. 4B: graphical representation of fluorescence signal depending on the concentration of the calicivirus RdRp and shown for different reaction times as indicated.
- Fig. 5: shows schematic representations of a further RNA detection method illustrating a fluorescence resonance energy transfer assay (FRET) using the calicivirus RNA-dependent RNA polymerase (RdRp) and a single stranded RNA as the template in the presence of labeled DNA oligoprobes having a fluorescence donor (D) and a second oligoprobe coupled to a fluorescence quencher (Q). Fig. 5A: an ssRNA-DNA hybrid is generated by hybridizing the single-stranded RNA to a labeled DNA oligoprobe having a fluorescence donor at its 5'-end and a second DNA oligoprobe containing a fluorescence quencher at its 3'-end. The oligoprobe containing a donor at its 5'-end is complementary to the 3'-terminus of the template RNA, and the oligoprobe containing the quencher is complementary to the 5'-terminus of the template RNA. Emission of energy by the donor is attenuated by the quencher through resonance energy transfer. Fig. 5B: schematic representation of strand displacement by the calicivirus RNA-dependent RNA polymerase (RdRp). The RdRp synthesizes RNA starting at the 3'-terminus of the template (ssRNA-Template), and displacing the oligoprobe-DNA-donor hybridized at the 3'-end of the ssRNA-template. RNA synthesis results in a double stranded RNA molecule. Strand displacement leads to release of the oligoprobe-DNA-donor and the Oligoprobe-DNA-quencher from the ssRNA-template, with subsequent detection of the fluorescence emitted by the donor.

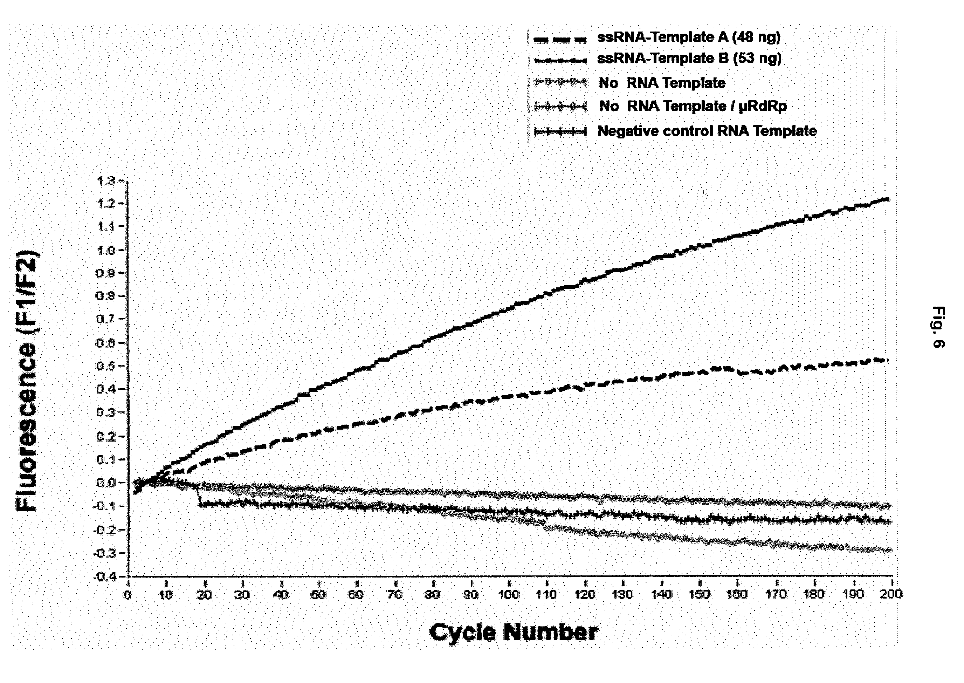

- Fig. 6: shows a graphical representation of fluorescence emission dependent on the number of reaction cycles which illustrates an example of fluorescence resonance energy transfer assay (FRET) using the calicivirus RNA-dependent RNA polymerase (RdRp) and a single stranded RNA as the template in the presence of a labeled DNA oligoprobe containing a fluorescence donor and a second oligoprobe having a fluorescence. In this experiment, two different ssRNA templates (template A and template B) in different amounts (ng as indicated) were used. Template B is miR-375 and has the sequence (5'-UUUGUUCGUUCGGCUCGCGUGA-3', SEQ ID NO: 9). Template A is modified form of miR-375 having the sequence 5'-UUUGUUCGUUCGGCUCGCGUGACCC-3' (SEQ ID NO: 10). µRdRp: active site mutant of calicivirus RdRp. Negative control RNA template: ssRNA having no complementarity to the oligoprobes used.

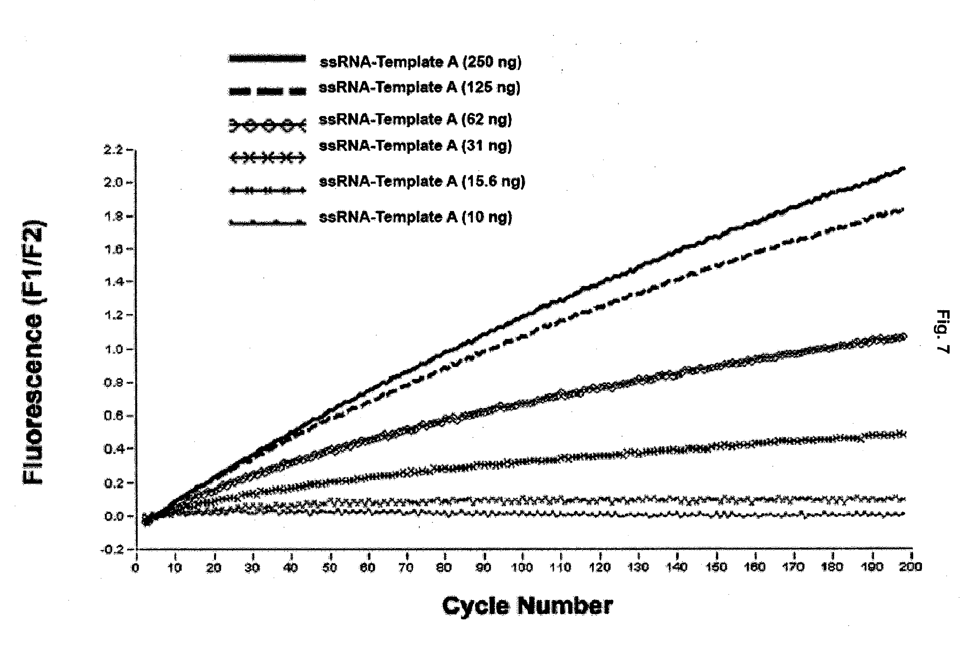

- Fig. 7: shows a graphical representation of fluorescence emission dependent on the number of reaction cycles which illustrates the dependency of the synthesis of dsRNA through strand displacement by the calicivirus RdRp on the template concentration. Increasing amounts of template A (from 10 to 250 ng, as indicated) were used. Template A is the same as described in Fig. 6.

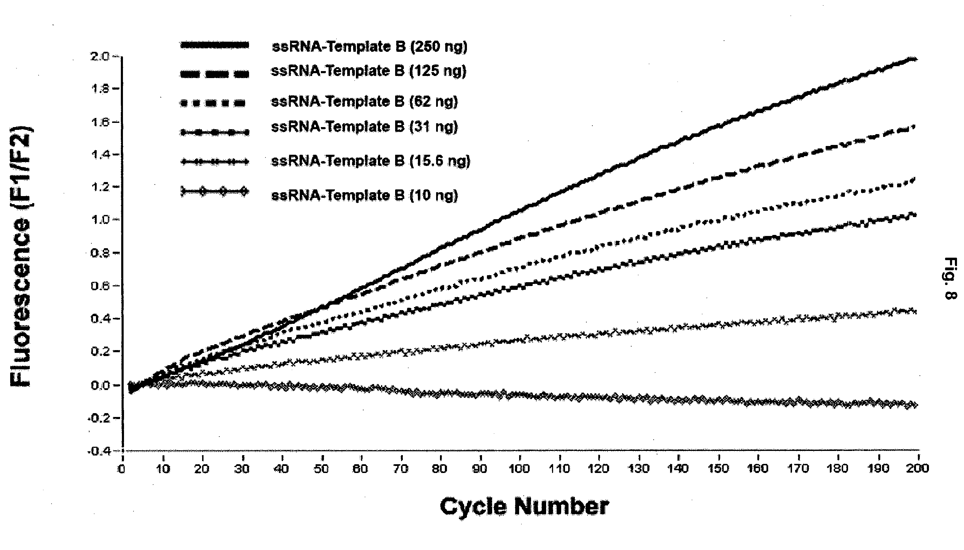

- Fig. 8: shows a further graphical representation of fluorescence emission dependent on the number of reaction cycles which illustrates the dependency of the synthesis of dsRNA through strand displacement by the calicivirus RdRp on the template concentration. Increasing amounts of template B (from 10 to 250 ng, as indicated) were used. Template B is the same as described in Fig. 6.

The present invention is further illustrated by the following non-limiting examples.

### EXAMPLES

### Example 1: Strand displacement assay using calicivirus RdRp

The reaction mix (25 µl) contains the RdRp (sapovirus; 6 µM), the Q/D-dsRNA-hybrid at a final concentration of 4 µM, the RdRp-Buffer (consisting of HEPES pH 8.0 4 mM, MnCl₂ 0.6 mM, ammonium acetate 25 mM, DTT 0.25 mM), ATP, UTP, CTP, GTP (designed as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is shown in Fig. 2A. The increase of fluorescence Fig. 2A, (filled line) was observed in a reaction consisting of the Calicivirus RdRp, the ribonucleotide triphosphates (rNTP), the RdRp buffer, and the Q/D-dsRNA hybrid as mentioned previously. As a control, the same reaction was run in the absence of rNTP (Fig. 2A, dashed line).

The products of the reactions were analyzed using native polyacrylamide gel electrophoresis (PAGE). The RdRp synthesizes a double-stranded RNA product only in the presence of all components of the reaction as mentioned above. Synthesis of the dsRNA occurs by strand displacement, leading to a double-stranded RNA molecule consisting of the single-stranded RNA labelled at its 5'-terminus with the quencher (ssRNA-Q) and its complementary single-stranded RNA synthesized by the RdRp, as shown in Fig. 2B. When rNTPs are omitted, no dsRNA is synthesized, as shown Fig. 2B as well. The reaction educt used in both reactions is denoted in Fig. 2B as "Q/D-dsRNA-Hybrid".

### Example 2: Strand displacement does not occur with active-site mutant of calicivirus RdRp

The same reactions were carried out as in Example 1, but using an active site mutant of the sapovirus RdRp (µRdRp) in a control reaction. The results are shown in Fig. 3.

Thus, the reaction mix (25 µl) contains the RdRp (6 µM), the Q/D-dsRNA-Hybrid at a final concentration of 4 µM, the RdRp buffer (consisting of HEPES pH 8.0 4 mM, MnCl₂ 0.6 mM, ammonium acetate 25 mM, DTT 0.25 mM), ATP, UTP, CTP, GTP (here designed as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is monitored. The increase of fluorescence (filled line) was observed in a reaction containing the sapovirus RdRp, the ribonucleotide triphosphates (rNTP), the RdRp-Buffer, and the Q/D-dsRNA hybrid as mentioned previously. As a control, the same reaction was run in the presence of an active site mutant of the RdRp (µRdRp) leading to total inhibition of RNA synthesis by the enzyme (dashed line in Fig. 3A).

The products of the reactions were analyzed using native polyacrylamide gel electrophoresis (PAGE). Synthesis of the dsRNA occurs by strand displacement, leading to a double-stranded RNA molecule consisting of the single-stranded RNA labelled at its 5'-terminus with the quencher (ssRNA-Q) and its complementary single-stranded RNA synthesized by the sapovirus RdRp, as indicated in Fig. 3B. When the µRdRp is used, no dsRNA is synthesized, as shown in Fig. 3B. The reaction educt used in both reactions is the Q/D-dsRNA hybrid.

### Example 3: Concentration and time dependency of dsRNA synthesis through strand displacement by calicivirus RdRp.

The reaction mix contained increasing concentrations of sapovirus RdRp (2.5 µM, 3 µM, 4 µM, and 6 µM; as indicated), the Q/D-dsRNA-hybrid at a final concentration of 4 µM, the RdRp buffer (HEPES pH 8.0 4 mM, MnCl₂ 0.6 mM, ammonium acetate 25 mM, DTT 0.25 mM), ATP, UTP, CTP, GTP (here designated as ribonucleotides triphosphates, rNTP) at a final concentration of 0.4 mM each. The quencher used is TAMRA, and the donor is 6-FAM. Fluorescence is monitored in real-time on the LightCycler 1.5 machine (Roche, Basel, Switzerland). Each cycle corresponds to a 15 seconds incubation of the reaction at 30°C, with subsequent measurement of fluorescence emission. The increase of the fluorescence over a period of 3000 seconds (200 cycles x 15 seconds = 3000 seconds) is shown in Fig. 4A for all four different concentrations of the calicivirus RdRp.

For elucidating the time-dependency of RNA synthesis by the calicivirus RdRp through strand-displacement, the reaction mix (25 µl) contained increasing concentrations of sapovirus RdRp (2.5 µM, 3 µM, 4 µM, and 6 µM), the Q/D-dsRNA-hybrid at a final concentration of 4 µM, the RdRp-Buffer (consisting of HEPES pH 8.0 4 mM, MnCl₂ 0.6 mM, ammonium acetate 25 mM, DTT 0.25 mM), ATP, UTP, CTP, GTP (here designed as ribonucleotides triphosphates, rNTP) at a final concentration of 1 mM each. The quencher used is TAMRA, and the donor is 6-FAM. Fluorescence is monitored in real-time on the LightCycler 1.5 machine at 10 min, 25 in and 50 min, as indicated in Fig. 4B. Fig. 4B demonstrates that the increase of the fluorescence is dependent of the RdRp concentration and the reaction time.

### Example 4: RNA strand displacement assay using two oligonucleotide probes

The reaction mix (25 µl) contains the RdRp (sapovirus; 7.5 µM), a quencher-oligoprobe (Q) and a donor-oligoprobe (D) at a final concentration of 0.5 µM, the RdRp buffer (containing HEPES pH 8.0 4 mM, MnCl₂ 0.6 mM, ammonium acetate 25 mM, DTT 0.25 mM), ATP, UTP, CTP (designated as ribonucleotides triphosphates, rNTP) at a final concentration of 0.4 mM each and GTP at a concentration of 2 mM. The fluorescence quencher used is TAMRA, and the fluorescence donor is 6-FAM. Fluorescence is monitored in real-time on the LightCycler 1.5 machine (Roche, Basel, Switzerland). Each cycle corresponds to a 15 seconds incubation of the reaction at 30°C, with a subsequent measurement of fluorescence emission. The increase of the fluorescence over a period of 3000 seconds (200 cycles x 15 seconds = 3000 seconds) is measured. The results of the experiments are shown in Fig. 6.

An increase of fluorescence was observed in a reaction containing the calicivirus RdRp, the ribonucleotide triphosphates (rNTPs), RdRp-buffer, the quencher-oligoprobe (Q), the donor-oligoprobe (D) and either 53 ng of template B (miR-375: 5'-UUUGUUCGUUCGGCUCGCGUGA-3', SEQ ID NO: 8) or 48 ng of template A (modified sequence of miR-375: 5'-UUUGUUCGUUCGGCUCGCGUGACCC3', SEQ ID NO: 9). As a control, the same reaction was run in the presence of an active site mutant of the RdRp (µRdRp) leading to total inhibition of RNA synthesis by the enzyme (µRdRp), and subsequent absence of fluorescence signal. When no RNA template is present in the reaction, or a negative control ssRNA-template not complementary to the quencher-oligoprobe (Q) and the donor-oligoprobe (D) is used, no signal is detected.

### Example 5: Dependency of strand displacement assay on template concentration

The reaction mix (25 µl) contains the RdRp (sapovirus; 7.5 µM), the quencher-oligoprobe (Q) and the donor-oligoprobe (D) at a final concentration of 0.5 µM, the RdRp-Buffer (consisting of HEPES pH 8.0 4 mM, MnCl₂ 0.6 mM, ammonium acetate 25 mM, DTT 0.25 mM), ATP, UTP, CTP (here designated as ribonucleotides triphosphates, rNTP) at a final concentration of 0.4 mM each and GTP at concentration of 2 mM. The quencher used is TAMRA, and the donor is 6-FAM. Fluorescence is monitored in real-time on the LightCycler 1.5 machine (Roche, Basel, Switzerland). The reactions contain different concentrations of template A or template B (10 ng, 15.6 ng, 31 ng, 62 ng, 125 ng, 250 ng). Each cycle corresponds to a 15 seconds incubation of the reaction mixture at 30°C, with subsequent measurement of fluorescence emission. The increase of the fluorescence over a period of 3000 seconds (200 cycles x 15 seconds = 3000 seconds) is measured. The increase of fluorescence was observed in a reaction containing the calicivirus RdRp, the ribonucleotide triphosphates (rNTP), RdRp-buffer, and the quencher-oligoprobe (Q) and the donor-oligoprobe (D).

As shown in Fig. 7 and Fig. 8, a detectable increase in fluorescence emission is observed at template concentrations above 10 ng (template B) or 15.6 ng (template A), respectively.

## Claims

1. A kit for detecting a target RNA sequence in a sample comprising:
- at least one labelled oligonucleotide containing a sequence substantially complementary to a region of the target RNA sequence wherein the at least one oligonucleotide is blocked at its 3'-end;
- an RNA-dependent RNA polymerase (RdRp) of a virus of the *Caliciviridae* family having an RNA duplex separation activity under polymerisation conditions and being capable of *de novo* RNA synthesis in the absence of a primer.

2. The kit of claim 1 wherein the oligonucleotide contains a fluorescent label.

3. The kit of claim 2 wherein the oligonucleotide contains a fluorescent label at its 5'-end.

4. The kit according to any one of the preceding claims wherein the labelled oligonucleotide hybridises to the 5'-end of the target RNA.

5. The kit of claim 4 further comprising a second oligonucleotide containing a sequence substantially complementary to a second region of the target RNA sequence which does not overlap with the region of complementarity of the labelled oligonucleotide and which second oligonucleotide contains a chemical moiety capable of quenching the fluorescence of the fluorescent label of the labelled oligonucleotide when both the labelled and the second oligonucleotide are hybridised to the target RNA sequence.

6. The kit according to any one of the preceding claims further containing a quencher molecule capable of quenching the fluorescence of the label of the oligonucleotide when hybridised to the target RNA.

7. The kit according to any one of the preceding claims wherein the labelled oligonucleotide and, optionally, the second oligonucleotide has (have) a length of from 5 to 20 nucleotides.

8. The kit of claim 7 wherein the labelled oligonucleotide and, optionally, the second oligonucleotide has (have) a length of from 10 to 12 nucleotides.

## Patentansprüche

1. Kit zur Detektion einer Ziel-RNA-Sequenz in einer Probe, umfassend:
- mindestens ein markiertes Oligonukleotid, das eine Sequenz enthält, die im Wesentlichen komplementär zu einem Abschnitt der Ziel-RNA-Sequenz ist, wobei das mindestens eine Oligonukleotid an seinem 3'-Ende blockiert ist;
- eine RNA-abhängige RNA-Polymerase (RdRp) eines Viruses der Familie der *Caliciviridae,* die eine RNA-Duplex-Trennungsaktivität unter Polymerisierungsbedingungen aufweist und zur De-novo-RNA-Synthese in Abwesenheit eines Primers befähigt ist.

2. Kit nach Anspruch 1, wobei das Oligonukleotid eine Fluoreszenzmarkierung enthält.

3. Kit nach Anspruch 2, wobei das Oligonukleotid eine Fluoreszenzmarkierung an seinen 5'-Ende enthält.

4. Kit nach einem der vorhergehenden Ansprüche, wobei das markierte Oligonukleotid mit dem 5'-Ende der Ziel-RNA hybridisiert.

5. Kit nach Anspruch 4, das weiter ein zweites Oligonukleotid umfasst, welches eine Sequenz enthält, die im Wesentlichen komplementär zu einem zweiten Abschnitt der Ziel-RNA-Sequenz ist, die nicht mit dem Abschnitt der Komplementarität des markierten Oligonukleotids überlappt und wobei das zweite Oligonukleotid eine chemische Einheit enthält, die zum Quenchen der Fluoreszenzmarkierung des markierten Oligonukleotids befähigt ist, wenn sowohl das markierte als auch das zweite Oligonukleotid mit der Ziel-RNA-Sequenz hybridisiert sind.

6. Kit nach einem der vorhergehenden Ansprüche, das weiter ein Quenchermolekül enthält, das zum Quenchen der Fluoreszenz der Markierung des Oligonukleotids befegt ist, wenn es mit der Ziel-RNA hybridisiert ist.

7. Kit nach einem der vorhergehenden Ansprüche, wobei das markierte Oligonukleotid und gegebenenfalls das zweite Oligonukleotid eine Länge von 5 bis 20 Nukleotiden aufweist/aufweisen.

8. Kit nach Anspruch 7, wobei das markierte Oligonukleotid und gegebenenfalls das zweite Oligonukleotid eine Länge von 10 bis 12 Nukleotiden aufweist/aufweisen.

## Revendications

1. Kit de détection d'une séquence d'ARN cible dans un échantillon, comprenant:
- au moins un oligonucléotide marqué contenant une séquence sensiblement complémentaire à une région de la séquence d'ARN cible dans laquelle ledit au moins oligonucléotide est bloqué à son extrémité 3',
- une ARN polymérase ARN-dépendante (RdRp) d'un virus de la famille des *Caliciviridae* ayant une activité de séparation des duplex d'ARN dans des conditions de polymérisation, et étant capable de synthétiser l'ARN *de novo* en l'absence d'une amorce.

2. Kit selon la revendication 1, dans lequel l'oligonucléotide contient un marqueur fluorescent.

3. Kit selon la revendication 2, dans lequel l'oligonucléotide contient un marqueur fluorescent à son extrémité 5'.

4. kit selon l'une quelconque des revendications précédentes, dans lequel l'oligonucléotide marqué s'hybride à l'extrémité 5' de l'ARN cible.

5. Kit selon la revendication 4, comprenant en outre un second oligonucléotide contenant une séquence sensiblement complémentaire à une seconde région de la séquence d'ARN cible qui ne recouvre pas la région complémentaire de l'oligonucléotide marqué et qui contient un groupement chimique capable d'éteindre la fluorescence du marqueur fluorescent de l'oligonucléotide marqué lorsque à la fois l'oligonucléotide marqué et le second oligonucléotide sont hybridés à la séquence d'ARN cible.

6. Kit selon l'une quelconque des revendications précédentes, contenant en outre une molécule extinctrice capable d'éteindre la fluorescence du marqueur de l'oligonucléotide lorsqu'il est hybridé à l'ARN cible.

7. Kit selon l'une quelconque des revendications précédentes, dans lequel l'oligonucléotide marqué et, optionnellement, le second oligonucléotide a (ont) une longueur de 5 à 20 nucléotides.

8. Kit selon la revendication 7, dans lequel l'oligonucléotide marqué et, optionnellement, le second oligonucléotide a (ont) une longueur de 10 à 12 nucléotides.
